# EUROPÄISCHE PATENTSCHRIFT

(11) **EP 2 253 265 B1**
(45) Veröffentlichungstag und Bekanntmachung des Hinweises auf die Patenterteilung: **10.04.2013**
(21) Anmeldenummer: 10005205.9
(22) Anmeldetag: 19.05.2010
(51) Int. Cl.: A61B 1/267, A61B 1/00, A61B 1/005, A61B 1/012, G02B 23/26, G02B 23/24

(54) **Endoskop**
Endoscope
Endoscope

(30) Priorität: 20.05.2009 DE 102009022118
(43) Veröffentlichungstag der Anmeldung: 24.11.2010
(73) Patentinhaber: Karl Storz GmbH & Co. KG, 78532 Tuttlingen (DE)
(72) Erfinder: Eisele, Hans-Peter, 78532 Tuttlingen (DE)
(74) Vertreter: Fugmann, Winfried

(56) Entgegenhaltungen:
- DE-A1- 10 351 185
- US-A- 4 838 245
- US-A1- 2004 093 906
- US-A1- 2005 090 712

## Beschreibung

Die vorliegende Erfindung betrifft ein Endoskop, insbesondere ein Endoskop zur Intubation eines Atemwegs, nach dem Oberbegriff von Anspruch 1.

Endoskope dieser Art werden insbesondere in der Anästhesie oder Notfallmedizin zur Intubation eines Atemwegs eines Menschen verwendet. Hierbei wird ein Intubationsschlauch durch die Luftröhre eingeführt, um den Patienten mit Luft bzw. Sauerstoff zu versorgen. Insbesondere bei Patienten mit anatomisch bedingt schwieriger Intubation ist es notwendig, die korrekte Einführung des Intubationsschlauchs zu kontrollieren. Hierfür wird der Schaft des Endoskops durch die Nase oder den Mund des Patienten eingeführt. Um dem Arzt beim fortschreitenden Einführen des Endoskopschafts stets eine optimale Position zu ermöglichen, ist ein abwinkelbares Okularteil vorgesehen. Zur Übertragung des endoskopischen Bilds vom distalen (d. h. dem Arzt abgewandten) zum proximalen (dem Arzt zugewandten) Ende des Endoskops wird in gattungsgemäßen Endoskopen ein flexibler oder semiflexibler Bildleiter verwendet, der in der Regel aus einem Bündel von Lichtleitfasern besteht.

Aus US 4,838,245 ist ein derartiges Endoskop bekannt, das einen langerstreckten Schaft aufweist, der zur Einführung in eine Körperhöhle in geeigneter Weise gebogen werden kann. Mit dem Schaft ist ein Kopfteil verbunden, mit dem über ein Gelenk abwinkelbar ein Okularteil verbunden ist. Ein flexibler Bildleiter, der zur Übertragung des Bilds vom distalen Ende des Schafts zum Okular dient, ist durch das Gelenk geführt und ermöglicht eine Abwinkelung des Okularteils. Auf diese Weise kann der Arzt bei der Einführung des Endoskops in den Körper des Patienten auf einfache Weise der veränderlichen Orientierung des Endoskopschafts folgen. Eine Drehung des Endoskops um die Längsachse wird durch das Gelenk ungehindert übertragen.

In US 5,377,668 wird ein faseroptisches Endoskop offenbart, bei dem ein oder mehrere schwenkbare Okularteile vorgesehen sind. Der Bildleiter kann in einen nichtwiederverwendbaren Schaft eingesetzt werden, worin er zur Realisierung unterschiedlicher Blickrichtungen beweglich angeordnet ist.

Gemäß EP 1 859 728 A2 weist ein Endoskop einen Schaft und ein Okular auf, wobei das Okular am proximalen Ende eines Endoskopkopfes angeordnet ist. Zwischen dem Schaft und dem Okular ist ein Gelenk vorgesehen zum Abwinkeln des Okulars relativ zu einer Längsrichtung des Schafts. Dabei ist das Gelenk derart ausgebildet, dass das Okular in zwei Ebenen abwinkelbar ist. Hierfür kann das Gelenk insbesondere in Form eines Kugelgelenks oder eines Kardangelenks ausgebildet sein. Der Bildleiter kann im Bereich des Gelenks in einem Schutzschlauch aufgenommen sein, der den Bildleiter vor Beschädigungen durch das Gelenk schützt.

Aus DE 103 51 185 A1 ist ein heißdampfsterilisierbares Endoskop mit einem Gehäuse bekannt, das in seinem distalen Endbereich als gerades Schaftrohr ausgebildet ist und in seinem proximalen Endbereich einen Biegeabschnitt mit sich daran anschließendem starren proximalen Endstück aufweist. Das Gehäuse ist von einem Bildleiter durchlaufen, der im Schaftrohr von einem starren Hüllrohr umgeben ist, das vor dem Objektiv mit einem Fenster geschlossen ist. Das Hüllrohr endet distalseitig des Biegeabschnitts. Im Bereich des Biegeabschnitts ist der Bildleiter von einem biegeflexiblen Metallbalg umgeben, der proximal am Endstück und distal am proximalen Ende des Hüllrohrs dampfdicht befestigt ist. Das Gehäuse selbst ist nicht dampfdicht ausgebildet.

Aus der US 2005/0090712 A1 ist ein Intubationsendoskop bekannt, das an seinem proximalen Ende einen Biegeabschnitt mit Okular aufweist. Ein Lichtleiter ist in einem vom proximalen zum distalen Ende des Instruments verlaufenden Durchgang geführt.

Der in den genannten Endoskopen zur Übertragung des endoskopischen Bilds dienende Bildleiter besteht in der Regel aus einem Bündel von Lichtleitfasern, die sowohl gegen mechanische Beschädigung als auch gegen heißen Dampf geschützt werden müssen. Dabei ist es insbesondere im Bereich der Abwinkelung notwendig, den Bildleiter vor Beschädigung durch Reibung bei der Abwinkelung zu schützen, beispielsweise durch Reibung mit der Innenseite eines den Bildleiter umgebenden Metallbalgs, wobei der Bildleiter insbesondere durch die Wellstruktur des Metallbalgs reibgeschädigt werden kann. Ebenso sind Bildleiter in der Regel empfindlich gegen heißen Dampf, der bei der Heißdampfsterilisation eindringen und beispielsweise zu Glaskorrosion führen kann. Darüber hinaus kann in ein optisches System eingedrungene Feuchtigkeit zum Beschlagen optischer Elemente führen und dadurch die Funktion beeinträchtigen. Deshalb sind Schutzmaßnahmen gegen das Eindringen von Heißdampf, auch unter den bei der Heißdampfsterilisation verwendeten Druckbedingungen, erforderlich, insbesondere im Bereich der Abwinkelung.

Es ist die Aufgabe der vorliegenden Erfindung, ein Endoskop der genannten Art anzugeben, das einen verbesserten Schutz gegen die erwähnten Schadeinflüsse aufweist.

Diese Aufgabe wird erfindungsgemäß durch ein Endoskop mit den Merkmalen des Anspruchs 1 gelöst.

Ein erfindungsgemäßes Endoskop umfasst einen Schaft, der zur Einführung in einen Hohlraum, insbesondere einen körperinneren Hohlraum eines menschlichen oder tierischen Körpers, ausgebildet ist. Der Schaft kann starr, flexibel oder semiflexibel ausgebildet sein.

Ist der Schaft starr oder semiflexibel, so kann der Schaft gerade oder auch zumindest abschnittsweise gekrümmt ausgebildet sein. Im Innern des Schafts ist ein Objektiv angeordnet, das zur Aufnahme eines Bildes eines zu beobachtenden Bereichs dient, beispielsweise eines Abschnitts eines Atemwegs. Zur Weiterleitung des vom Objektiv entworfenen Bildes ist im Schaft ein Bildleiter angeordnet, aus einem Bündel von Lichtleitfasern, insbesondere einem geordneten Bündel von Lichtleitfasern, besteht.

Ferner umfasst ein erfindungsgemäßes Endoskop einen Endoskopkopf, der ein erstes und ein zweites Kopfteil umfasst. Das erste Kopfteil ist mit dem Schaft verbunden. Hierbei kann das erste Kopfteil eine Längsachse aufweisen, die insbesondere mit der Längsachse des Schafts übereinstimmen kann. Ist der Schaft abschnittsweise gebogen ausgebildet, so kann die Längsachse des ersten Kopfteils insbesondere mit der Längsachse des proximalen Endbereichs des Schafts übereinstimmen.

Das zweite Kopfteil umfasst ein Okular zur Betrachtung des vom Bildleiter weitergeleiteten Bildes durch einen Beobachter, insbesondere durch den Arzt. Das zweite Kopfteil ist mit dem ersten Kopfteil verbunden und gegenüber dem ersten Kopfteil abwinkelbar. Hierdurch wird es dem Arzt ermöglicht, unabhängig von der Ausrichtung des Endoskops stets eine optimale Körperhaltung einzunehmen. Dies ist insbesondere dann wichtig, wenn das Endoskop beim Einführen in eine Körperhöhle entlang eines gekrümmten Weges bewegt werden muss, beispielsweise weil die Körperhöhle selbst gekrümmt ist.

Der Bildleiter setzt sich vom Schaft durch das erste Kopfteil in das zweite Kopfteil fort. Der Bildleiter ist hierfür zumindest im Bereich der Verbindung zwischen dem ersten und dem zweiten Kopfteil soweit flexibel ausgebildet, dass er der Abwinkelung des zweiten Kopfteils, auch bei wiederholter Bewegung, folgen kann.

Erfindungsgemäß ist der Bildleiter von einem zumindest im Bereich der Verbindung zwischen dem ersten und dem zweiten Kopfteil biegeflexiblen Hüllrohr umgeben, das vom Schaft bis zum zweiten Kopfteil durchgehend ausgebildet ist. Dadurch, dass das Hüllrohr biegeflexibel ausgestaltet ist, wird die Abwinkelung des zweiten Kopfteils gegenüber dem ersten Kopfteil ermöglicht. Ein durchgehendes Hüllrohr bietet einen bestmöglichen Schutz gegen mechanische Beschädigungen durch Reibung mit weiteren Bauelementen, die insbesondere im Bereich der Verbindung zwischen erstem und zweitem Kopfteil in Kontakt mit dem Bildleiter gelangen können. Schließlich wird dadurch, dass der Bildleiter von einem durchgehenden Hüllrohr umgeben ist, insbesondere von einem dampfdichten durchgehenden Hüllrohr, auf einfache Weise ein dampfdichter Abschluss des Bildleiters gegenüber der Umgebung erzielt. Insbesondere ist der Bildleiter dadurch im Bereich der Verbindung zwischen erstem und zweitem Kopfteil gegen das Eindringen von Heißdampf beim Sterilisieren geschützt, ohne dass in diesem Bereich weitere Abdichtungsmaßnahmen getroffen werden müssen.

Das Hüllrohr ist dabei insbesondere zwar biegeflexibel, aber steif gegen seitliche Druckkräfte ausgebildet. Hierdurch werden sowohl eine Verformung des Hüllrohrs beim Einwirken von Druckkräften beim Sterilisieren als auch Beschädigungen des Bildleiters beim Biegen des Hüllrohrs oder durch Kontakt mit anderen Bauelementen beim Abwinkeln des zweiten Kopfteils vermieden.

Als Materialien für das Hüllrohr kommen insbesondere ausreichend verformbare metallische Werkstoffe in Frage, beispielsweise NiTi-Legierungen (Nitinol). Solche metallischen Hüllrohre weisen eine ausgezeichnete Dichtheit gegenüber Heißdampf auf und ebenso eine ausreichende Steifigkeit gegen seitlichen Druck. Werden etwas geringere Anforderungen an die Dichtheit gegenüber Heißdampf gestellt, können auch Hüllrohre aus bestimmten Kunststoffen verwendet werden, die ausreichende thermische und mechanische Stabilität aufweisen und für die Verwendung in Endoskopen zugelassen sind, beispielsweise Polyetheretherketon (PEEK). Ebenso können auch mehrlagige Hüllrohre verwendet werden, die beispielsweise unterschiedliche Schichten zur Gewährleistung der Dampfdichtheit und der mechanischen Stabilität aufweisen.

In bevorzugter Weise ist das Hüllrohr im Bereich der Verbindung zwischen erstem und zweitem Kopfteil derart dimensioniert, dass der Bildleiter bei der Abwinkelung nicht oder nur in geringem Maße in Kontakt mit der Innenwand des Hüllrohrs kommt. Hierdurch wird eine Beschädigung durch Reibung auch bei vielfachen Abwinkelungsbewegungen sowie bei der weiteren Handhabung des Endoskops vermieden. Weiterhin ist die Innenwand des Hüllrohrs bevorzugt derart ausgebildet, dass auch bei einem eventuellen Kontakt mit dem Bildleiter die Reibung möglichst gering bleibt. Hierfür kann die Innenwand insbesondere glatt, also ohne eine Wellenstruktur bzw. mit möglichst geringer Rauhigkeit ausgebildet oder mit einer reibungsmindernden Beschichtung belegt sein, beispielsweise Polytetrafluorethylen (PTFE). Ebenso kann der Bildleiter innerhalb des Hüllrohrs auch von einem reibungsmindernden Schutzschlauch, etwa aus PTFE, umgeben sein.

Das Hüllrohr ist im Bereich der Verbindung zwischen erstem und zweitem Kopfteil von einem Metallbalg umgehen. In diesem Bereich muss eine Biegung des Bildleiters und damit eine Biegung des Hüllrohrs erfolgen können, um die Abwinkelung des zweiten Kopfteils zu ermöglichen. Dadurch, dass in dem Abschnitt des Hüllrohrs, in dem die Abwinkelung erfolgt, ein biegeflexibler Metallbalg vorhanden ist, wird das Hüllrohr und damit zusätzlich der Bildleiter gegen mechanische Beschädigungen bei der Abwinkelung geschützt. Darüber hinaus kann der Metallbalg ein Einknicken des Hüllrohrs bei starker Abwinkelung oder bei auch bei sehr häufiger Abwinkelung verhindern. Hierfür kann der Metallbalg eine Zwangsführung des Hüllrohrs darstellen. Zur Verminderung von Reibung kann zwischen Metallbalg und Hüllrohr beispielsweise ein Schlauch aus PTFE vorgesehen sein.

Der Metallbalg ist in seinem distalen Endbereich dampfdicht mit einem Gehäuse des ersten Kopfteils verbunden. Insbesondere ist das erste Kopfteil mit einem dampfdichten Gehäuse versehen, das dampfdicht mit dem Schaft verbunden oder gegen den Schaft abgedichtet sein kann. Zur Erzielung einer dampfdichten Verbindung zwischen Metallbalg und Gehäuse kann ein zylindrischer distaler Endabschnitt des Metallbalgs in eine entsprechende Bohrung des Gehäuses eingesetzt und beispielsweise durch Kleben, Schweißen oder Löten dicht mit dem Gehäuse verbunden sein.

Dadurch kann eine zusätzliche Sicherheit gegen das Eindringen von Heißdampf bei der Sterilisation erzielt werden. Auf diese Weise ist bereits der Innenraum des Gehäuses weitgehend dampfdicht ausgebildet, so dass die Anforderungen an die Dichtheit des Hüllrohres abgesenkt werden können. Insbesondere kann in diesem Fall auch ein Hüllrohr verwendet werden, das zumindest abschnittsweise aus einem Kunststoff, wie etwa PEEK, besteht, der gegenüber metallischen Werkstoffen einen geringeren Grad an Dampfdichtheit aufweist. Der Übergang zwischen den beiden Teilen des Hüllrohrs kann beispielsweise innerhalb des ersten Kopfteils angeordnet sein. Hierdurch ist eine besonders flexible Ausführung des Hüllrohrs im Bereich der Abwinkelung möglich.

Gemäß einer bevorzugten Ausführungsform schließt das Hüllrohr das Objektiv zumindest teilweise ein. In diesem Fall ist das Hüllrohr bis in die Nähe des distalen Endes des Schafts durchgeführt und ragt über das distale Ende des Bildleiters hinaus. Das Objektiv, das in der Regel aus einer Anordnung mehrerer Linsen besteht, ist von dem über das distale Ende des Bildleiters hinausragenden Teil des Hüllrohrs umschlossen. Auf diese Weise wird eine verbesserte mechanische Stabilität der Anordnung aus Objektiv und Bildleiter erzielt.

Bevorzugt ist das Hüllrohr dampfdicht mit dem Umfang einer Objektivlinse, insbesondere der distalen Endlinse des Objektivs, oder mit der Umfangsfläche einer Fassung bzw. Hülse, die eine einzelne Linse oder das Objektiv als Ganzes umgibt, verbunden. Hierdurch wird ein dampfdichter Abschluss des Hüllrohres am distalen Ende erzielt, wodurch Bildleiter und Objektiv gegen das Eindringen von Dampf geschützt sind.

Ebenso kann das Hüllrohr das Okular, das in der Regel aus mehreren Linsen aufgebaut ist, umschließen. In diesem Fall ragt das Hüllrohr über das proximale Ende des Bildleiters hinaus. Das Okular, das in der Regel aus einer Anordnung mehrerer Linsen besteht, ist von dem über das proximale Ende des Bildleiters hinausragenden Teil des Hüllrohrs umschlosscn. Auf diese Weise wird eine verbesserte mechanische Stabilität der Anordnung aus Okular und Bildleiter erzielt.

Bevorzugt ist das Hüllrohr dampfdicht mit dem Umfang einer Okularlinse, insbesondere der proximalen Endlinse des Okulars, oder mit der Umfangsfläche einer Fassung bzw. Hülse, die eine einzelne Linse oder das Okular als Ganzes umgibt, verbunden. Hierdurch wird ein dampfdichter Abschluss des Hüllrohres am proximalen Ende erzielt, wodurch Bildleiter und Okular gegen das Eindringen von Dampf geschützt sind.

Sind sowohl das distale als auch das proximale Ende des Hüllrohrs auf diese Weise dampfdicht abgeschlossen, so bildet das Hüllrohr eine dampfdichte Einheit, durch die der Bildleiter sowie Objektiv und Okular besonders sicher gegen das Eindringen von Dampf geschützt sind.

Gemäß einer weiteren bevorzugten Ausführungsform ist das Hüllrohr einteilig ausgebildet. In diesem Fall weist das Hüllrohr keine Verbindungsstellen auf, bei denen die Gefahr des Eindringens von Heißdampf bestehen könnte. Auf diese Weise wird eine besonders hohe Sicherheit gegen das Eindringen von Heißdampf erzielt, auch bei vielfacher Sterilisierung und bei vielfacher Abwinkelung des zweiten Kopfteils. Zudem ist die Konstruktion besonders einfach aufgebaut und ermöglicht eine kostengünstige Herstellung mit einer geringen Zahl an Fertigungsschritten.

Alternativ hierzu kann das Hüllrohr mehrteilig ausgebildet sein. So kann insbesondere das Hüllrohr im Bereich des Schafts aus einem anderen Material bestehen als im Bereich der Verbindung zwischen erstem und zweitem Kopfteil. Hierdurch ist es möglich, durch geeignete Wahl von Durchmesser, Wandstärke und Material die für den jeweiligen Bereich optimalen Eigenschaften zu erzielen. So kann beispielsweise im Bereich des Schafts eine hohe mechanische Stabilität, auch unter der durch die Anwendung bedingten Vorgabe eines geringen Durchmessers, wünschenswert sein, während im Bereich der Verbindung zwischen erstem und zweitem Kopfteil eine ausreichende Verformbarkeit, auch bei größerem Durchmesser, zur Ermöglichung der Abwinkelung gewünscht ist.

In bevorzugter Weise ist das Hüllrohr derart mehrteilig ausgebildet, dass es im Bereich des Schafts aus einem metallischen Material mit hoher Steifigkeit und guter Möglichkeit, es durch Schweißen, Löten, Kleben oder Schrumpfen mit einem Paarungspartner zu verbinden, besteht. Dies ermöglicht auch bei geringer Wandstärke eine Stabilisierung des Schafts und des Bildleiters. Das Hüllrohr kann in diesem Bereich eng am Bildleiter anliegen. Im Bereich der Verbindung zwischen erstem und zweitem Kopfteil weist das Hüllrohr demgegenüber einen größeren Durchmesser auf, so dass der Bildleiter möglichst wenig an der Innenwand der Hüllrohrs anliegt; das Hüllrohr ist in diesem Bereich aus einem Werkstoff ausgeführt, der eine ausreichende Biegsamkeit gewährleistet. Die unterschiedlichen Teile des Hüllrohrs sind dabei durch Kleben, Schweißen oder Löten miteinander möglichst dampfdicht verbunden.

Der proximale Endabschnitt des Metallbalgs kann mit dem Hüllrohr durch Kleben, Schweißen oder Löten dicht verbunden sein. In bevorzugter Weise ist der Metallbalg in seinem proximalen Endbereich jedoch dampfdicht mit einem Gehäuse des zweiten Kopfteils verbunden. Dieses ist ebenfalls dampfdicht ausgebildet. Diese Maßnahme hat den Vorteil, dass auch innerhalb des Gehäuses des zweiten Kopfteils bereits ein hohes Maß an Dampfdichtheit besteht, so dass nicht nur die Anforderungen an die Dampfdichtheit des Hüllrohrs in diesem Bereich abgesenkt werden können, sondern auch die im zweiten Kopfteil angeordneten optischen Elemente, insbesondere Okularlinsen und proximales Fenster, gegen das Eindringen von Feuchtigkeit geschützt sind.

Gemäß einer weiteren bevorzugten Ausführungsform ist das zweite Kopfteil über ein Gelenk mit dem ersten Kopfteil verbunden, das eine Abwinkelung des zweiten Kopfteils in einer Ebene zulässt. Hierdurch ist eine kontrollierte Abwinkelung möglich. Dies hat den weiteren Vorteil, dass nach einer Abwinkelung das zweite Kopfteil wieder sicher in die Ausgangsposition zurückgeführt werden kann, ohne dass eine mehrfache, schlangenförmige Biegung eines biegeflexiblen Abwinkelungsabschnitts oder ein dadurch verursachter Versatz des zweiten Kopfteils zurückbleiben. Diese Maßnahme dient daher einer kontrollierteren Handhabung sowie einer Erhöhung der Lebensdauer. Darüber hinaus ermöglicht ein Gelenk eine sicherere Übertragung von Drehmomenten, wenn eine Drehung des Endoskops um die Längsachse erforderlich ist, beispielsweise um eine gekrümmte Spitze des Endoskopschafts gemäß den anatomischen Gegebenheiten in eine Körperhöhle einzuführen oder um bei einer Seitblickoptik die Blickrichtung entsprechend den Anforderungen zu wählen.

In einer weiteren Ausgestaltung der Erfindung kann das Gelenk eine Abwinkelung des Kopfteils in mehreren Ebenen zulassen. Hierdurch wird eine besonders flexible Handhabung ermöglicht.

Gemäß einer weiteren bevorzugten Ausführungsform der Erfindung ist der mögliche Winkelbereich der Abwinkelung begrenzt. So kann beispielsweise bei einer Abwinkelung in einer Ebene der mögliche Winkelbereich auf einen Betrag von 45° oder bevorzugt 30°, gerechnet von einer gestreckten Anordnung des zweiten relativ zum ersten Kopfteil, begrenzt sein. Dies hat den Vorteil, dass übermäßige Abwinkelungen, die zu einer Beschädigung des Bildleiters oder des Hüllrohres führen können, sicher vermieden werden. Bei einer Abwinkelung in mehreren Ebenen kann eine solche Begrenzung in jeder Ebene oder auch in jeder beliebigen Raumrichtung, gerechnet von der Längsachse des ersten Kopfteils, bestehen.

In einer besonders bevorzugten Ausführungsform ist der Winkelbereich der Abwinkelung asymmetrisch zur Längsachse des ersten Kopfteils begrenzt. Da bei der Anwendung des erfindungsgemäßen Endoskops in der Regel eine Abwinkelung in einer Richtung bevorzugt wird, lässt sich hierdurch ohne wesentliche Einschränkung der Anwendungen der zulässige Winkelbereich verkleinern und dadurch die Gefahr einer Beschädigung, beispielsweise durch Ermüdung, verringern.

Insbesondere reicht es für die vorgesehene Anwendung des Endoskops in der Regel aus, dass eine Abwinkelung des zweiten Kopfteils aus einer gestreckten Position nur in einer Richtung möglich ist. Gerechnet von der Längsachse des ersten Kopfteils kann daher das zweite Kopfteil bzw. eine diesem zugeordnete Längsachse einen Winkelbereich von beispielsweise 0° bis 45° oder, in bevorzugter Weise, 0° bis 30° einnehmen. Ein derart verringerter Bewegungsbereich ermöglicht eine weitere Verlängerung der Lebensdauer des Endoskops, ohne die Anwendung wesentlich zu beeinträchtigen.

Gemäß einer weiteren bevorzugten Ausführungsform der Erfindung ist im Gelenk ein reibungserzeugendes Element vorgesehen. Durch den von diesem erzeugten Reibschluss kann es erreicht werden, dass das zweite Kopfteil eine vom Arzt einmal eingestellte Abwinkelung sicher beibehält, entgegen einer eventuellen Rückstellkraft des Bildleiters oder des Hüllrohrs und unabhängig von eventuelle einwirkenden Handhabungskräften oder der Schwerkraft. Erst durch Ausübung einer über eine Haltekraft hinausgehenden Kraft kann eine Änderung der Winkelposition bewirkt werden.

In bevorzugter Weise ist die Reibung einstellbar. Dies hat den Vorteil, dass für unterschiedliche Anwendungen die jeweils gewünschte Reibung eingestellt werden kann. Auch um unterschiedliche oder veränderliche Materialeigenschaften auszugleichen, kann eine Einstellbarkeit der Reibung vorteilhaft sein. Die Einstellung der Reibung kann ausschließlich bei der Herstellung erfolgen oder auch bei der Anwendung durch einen Benutzer möglich sein.

Das Gelenk kann insbesondere derart ausgebildet sein, dass mit einem der zueinander abwinkelbaren Kopfteile eine Achse verbunden ist, die in eine mit dem anderen Kopfteil verbundene Gelenkscheibe eingreift. Ist beispielsweise mit dem ersten Kopfteil eine quer zur Längsachse des ersten Kopfteils gerichtete Achse verbunden, so kann das zweite Kopfteil eine Gelenkscheibe aufweisen, in die die Achse eingreift. Die Achse kann dabei insbesondere auch durchbrochen bzw. mehrteilig ausgebildet sein, um eine ungehinderte Durchführung des Bildleiters mit umgebendem Hüllrohr zu ermöglichen. Hierfür kann die Gelenkscheibe eine Bohrung aufweisen. Diese Bohrung kann derart ausgeführt sein, dass die Gelenkscheibe bei zulässiger Abwinkelung nicht in Kontakt mit dem Hüllrohr bzw. einem dieses umgebenden Metallbalg kommt. In bevorzugter Weise ist die Bohrung exzentrisch angeordnet. Diese Maßnahme hat den Vorteil, dass mehr Raum für die Aufnahme weiterer Elemente, beispielsweise von Federn zur Erzeugung oder Einstellung der Reibung, vorhanden ist. Darüber hinaus ermöglicht die exzentrische Anordnung der Bohrung auch eine exzentrische Anordnung des Hüllrohrs bzw. des Metallbalgs, wodurch bei einem einseitigen Abwinkelungsbereich eine Minimierung der Reibung des Bildleiters mit dem Hüllrohr erreicht werden kann.

Ein erfindungsgemäßes Endoskop ist insbesondere zur Verwendung bei der Intubation eines Atemwegs geeignet. Der Anwendungsbereich der Erfindung ist jedoch nicht auf diese Anwendung beschränkt, vielmehr sind die Vorteile der Erfindung bei einer Vielzahl anderer endoskopischer Anwendungen nutzbar.

Weitere Aspekte der Erfindung ergeben sich aus der nachfolgenden Beschreibung eines bevorzugten Ausführungsbeispiels und der beigefügten Zeichnung. Es zeigen:
Fig. 1 eine Gesamtansicht eines erfindungsgemäßen Endoskop;
Fig. 2 den Endoskopkopf eines erfindungsgemäßen Endoskops, wobei eine gestreckte und eine abgewinkelte Position des zweiten Kopfteils dargestellt sind;
Fig. 3 eine teilweise aufgeschnittene Darstellung des Endoskopkopfs eines erfindungsgemäßen Endoskops;
Fig. 4 eine perspektivische, teilweise geöffnete Ansicht des Gelenks eines erfindungsgemäßen Endoskops;
Fig. 5 einen vergrößerten Längsschnitt durch das erste Kopfteil eines erfindungsgemäßen Endoskops;
Fig. 6 eine teilweise aufgeschnittene Darstellung eines Endoskopkopfes einer anderen Ausführungsform der Erfindung.

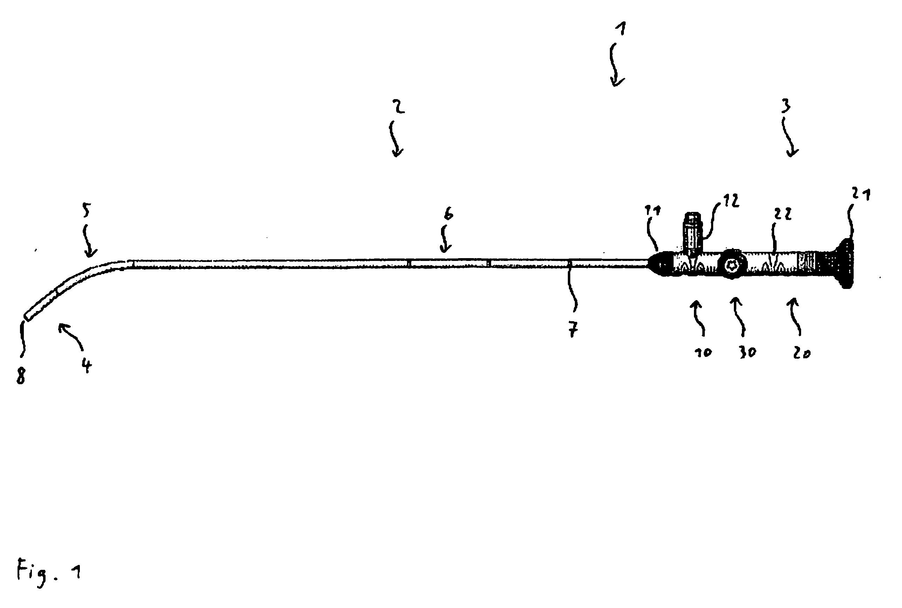

Gemäß Fig. 1 weist ein erfindungsgemäßes Endoskop 1, das auch als Endoskopoptik bezeichnet werden kann, einen langerstreckten Schaft 2 und einen Endoskopkopf 3 auf. Der Schaft ist zur Einführung in eine Körperhöhle ausgebildet und umfasst einen distalen Endbereich 4, der gerade ausgebildet sein kann, einen gekrümmten Abschnitt 5 und einen proximalen Abschnitt 6. Auf dem Schaft kann eine Mehrzahl von Markierungen 7 angebracht sein, die dem Arzt einen Anhaltspunkt geben, wie weit der Schaft bereits in die Körperhöhle eingeführt ist.

Der Endoskopkopf 3 weist ein erstes Kopfteil 10 und ein zweites Kopfteil 20 auf. Das erste Kopfteil 10 ist über einen Konus 11 mit dem proximalen Abschnitt 6 des Schafts 2 verbunden. Am ersten Kopfteil 10 setzt ein Lichtanschluss 12 an, an den ein Lichtleitkabel angesetzt werden kann zur Einleitung von in einer externen Lichtquelle erzeugtem Beleuchtungslicht, das zum distalen Ende 8 des Endoskops geführt wird und dort austritt. Es kann aber auch eine interne Lichtquelle, etwa im Endoskopkopf 3 oder im distalen Endbereich 4 des Schafts vorgesehen sein. Über ein Gelenk 30 ist das zweite Kopfteil 20 mit dem ersten Kopfteil 10 verbunden. Das zweite Kopfteil umfasst eine Okularmuschel 21 für einen visuellen Einblick durch einen Beobachter, insbesondere durch den Arzt. Die Okularmuschel kann auch für den Anschluss einer Videokamera ausgebildet sein, beispielsweise für Dokumentations- oder Schulungszwecke. Auf der Außenseite des ersten und des zweiten Kopfteils können Griffmulden 22 für eine bessere Handhabung angeordnet sein.

Der distale Endbereich 4 des Endoskops 1 enthält ein Objektiv (nicht dargestellt) zur Aufnahme eines endoskopischen Bilds eines Bildfelds, das sich, je nach Ausbildung des Objektivs, vor, schräg oder seitlich des distalen Endbereichs 4 des Endoskops befindet. Das von dem Objektiv aufgenommene Bild wird durch einen im Schaft 2, durch das erste Kopfteil 10 und durch das Gelenk 30 geführten Bildleiter (in Fig. 1 nicht dargestellt) zum zweiten Kopfteil geführt, wo das Bild über ein im zweiten Kopfteil 20 aufgenommenes Okular (nicht dargestellt) durch die Okularmuschel 21 betrachtet werden kann. Als distaler Lichteintritt bzw. als proximaler Lichtaustritt kann jeweils ein Fenster (nicht dargestellt) vorgesehen sein. Ferner kann das Endoskop 1 einen Arbeitskanal zur Einführung von Instrumenten sowie Kanäle und entsprechende Anschlüsse für Gase und Flüssigkeiten aufweisen (nicht dargestellt).

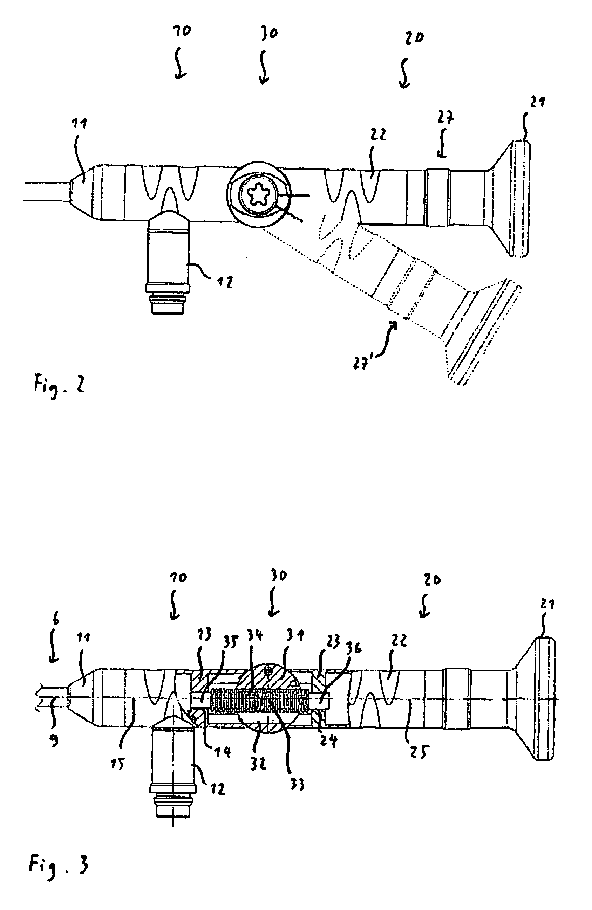

Wie in Fig. 2 dargestellt, kann das zweite Kopfteil 20 aus einer gestreckten Stellung 27 in eine abgewinkelte Stellung 27' geschwenkt werden. In bevorzugter Weise erfolgt die dabei in einer Ebene, die durch den proximalen Abschnitt 6, den gekrümmten Abschnitt 5 und den distalen Endabschnitt 4 des Schafts sowie den Lichtanschluss 12 vorgegeben wird, und zwar in Richtung auf den Lichtanschluss 12. Hierbei weist das Gelenk 30 bevorzugt einen solchen Betrag an Reibung auf, dass das zweite Kopfteil ohne weitere Krafteinwirkung in der abgewinkelten Stellung 27' verbleibt. Ebenso verbleibt das zweite Kopfteil in der gestreckten Stellung 27 sowie in jeder weiteren Position.

Fig. 3 zeigt das Gelenk 30 und die unmittelbar angerenzenden Bereiche des ersten und des zweiten Kopfteils in teilweise aufgeschnittener Darstellung. Das erste Kopfteil 10 weist ein Gehäuse 13 auf, das in Richtung auf das Gelenk 30 von einer zylindrischen Bohrung 14 durchbrochen ist. Das zweite Kopfteil umfasst ein Gehäuse 23, das in Richtung auf das Gelenk 30 eine zylindrische Bohrung 24 aufweist. Das erste Kopfteil 10 kann zumindest abschnittsweise zylindrisch ausgebildet sein und weist eine Längsachse 15 auf, die mit der Längsachse 9 des proximalen Schaftabschnitts 6 im Wesentlichen übereinstimmt. Das zweite Kopfteil 20 kann ebenfalls zumindest abschnittsweise zylindrisch ausgebildet sein. In der in Fig. 3 gezeigten gestreckten Position stimmt die Längsachse 25 des zweiten Kopfteils mit der Längsachse 15 des ersten Kopfteils im Wesentlichen überein. Die Bohrung 14 verläuft parallel, jedoch exzentrisch, d. h. nicht koaxial, zur Längsachse 15 des ersten Kopfteils 10, ebenso wie die Bohrung 24 exzentrisch zur Längsachse 25 des zweiten Kopfteils 20 verläuft.

Das Gelenk weist eine Gelenkscheibe 31 auf, die im Ausführungsbeispiel mit dem zweiten Kopfteil 20 verbunden und gegenüber dem ersten Kopfteil 10 bezüglich eines Drehpunkts 33 drehbar gelagert ist. Die Gelenkscheibe 31 ist durch eine bezüglich der Längsachse 25 des zweiten Kopfteils exzentrische Bohrung 32 durchbrochen, durch die ein biegeflexibler Metallbalg 34 geführt ist, der eine wellenförmige Struktur aufweist. Der Metallbalg 34 umfasst einen distalen Endbereich 35 und einen proximalen Endbereich 36. Der distale Endbereich 35 des Metallbalgs 34 ist in die Bohrung 14 eingesetzt und dort dampfdicht mit dem Gehäuse 13 verbunden, ebenso wie der proximale Endbereich 36 in die Bohrung 24 eingesetzt ist und dort dampfdicht mit dem Gehäuse 23 verbunden ist. Sowohl das Gehäuse 13 des ersten Kopfteils 10 als auch das Gehäuse 23 des zweiten Kopfteils 20 sind dampf dicht ausgeführt. Das Hüllrohr und der Bildleiter sind in Fig. 3 nicht dargestellt.

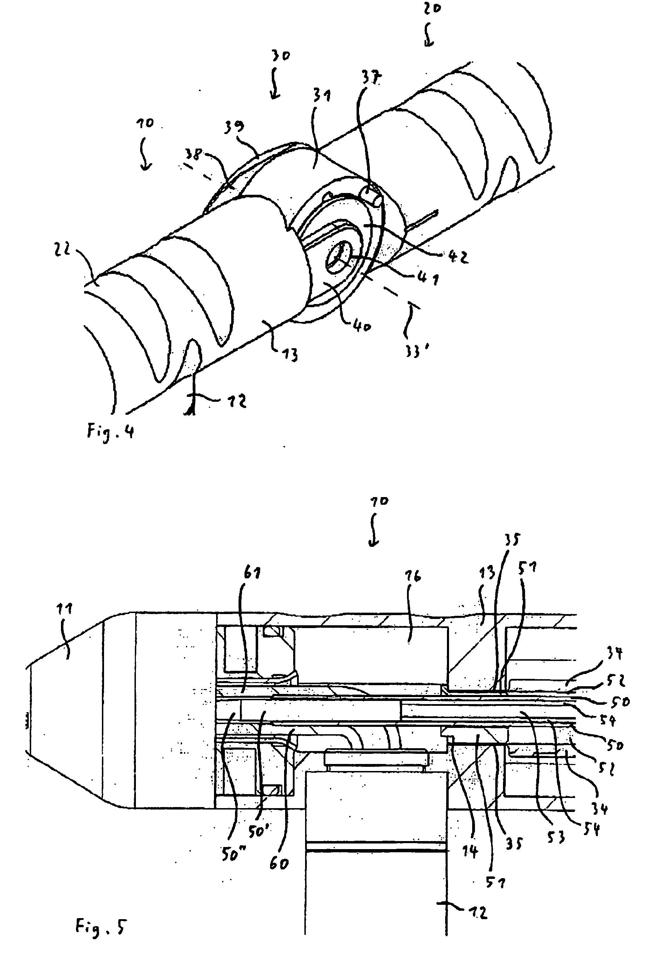

In Fig. 4 ist das Gelenk 30 teilweise geöffnet und zusammen mit den angrenzenden Bereichen des ersten und des zweiten Kopfteils perspektivisch dargestellt. Mit dem ersten Kopfteil 10 sind beidseitig des Gelenks zwei Scheiben 38 verbunden (in Fig. 4 ist nur die hintere Scheibe dargestellt), die auf der Außenseite zur besseren Handhabung Anfasungen 39 aufweisen. Ein Stift 37 greift in ein Langloch einer Scheibe 38 ein (nicht dargestellt), um den Schwenkbereich des zweiten Kopfteils zu begrenzen. Einteilig mit dem Gehäuse 13 des ersten Kopfteils sind Aufnahmen 40 ausgeführt, die Bohrungen 41 aufweisen, in die zur Festlegung der Drehachse 33' Schrauben (nicht dargestellt) eingesetzt sind, die in die Gelenkscheibe 31 eingreifen und durch die die von Gleitscheiben 42 vermittelte Reibung eingestellt werden kann.

Gemäß Fig. 5 weist der Faltenbalg 34 einen distalen Endabschnitt 35 auf, der in die Bohrung 14 des Gehäuses 13 eingesetzt ist und, beispielsweise durch Löten, Kleben, Schweißen oder Laserschweißen, mit diesem dampfdicht verbunden ist. Exzentrisch innerhalb des Metallbalgs ist ein Abschnitt 50 des Hüllrohrs angeordnet und durch Halter 51 gehalten. Der Zwischenraum 52 zwischen Metallbalg 34 und Hüllrohr 50 dient zur Ermöglichung der notwendigen Bewegung des Hüllrohrs relativ zum Metallbalg; zur Verminderung von Reibung kann beispielsweise ein Schlauch aus PTFE in den Zwischenraum 52 eingesetzt sein. Innerhalb des Hüllrohrs 50 und im Wesentlichen koaxial zur Längsachse 15 (nicht dargestellt) des ersten Kopfteils ist der aus einem Bündel von Lichtleitfasern bestehende Bildleiter 53 angeordnet. Der Zwischenraum 54 zwischen Hüllrohr und Bildleiter dient zur Verhinderung von Reibung zwischen Hüllrohr und Bildleiter und kann auch von einem Mittel zur Verminderung der Reibung, beispielsweise einem Schlauch aus PTFE, teilweise ausgefüllt sein.

Am ersten Kopfteil 10 ist weiterhin der Lichtanschluss 12 angesetzt, durch den aus Bündeln von Glasfasern bestehende Lichtleiter 60, 61 in den Innenraum 16 des Gehäuses 13 geführt werden. Die Lichtleiter 60, 61 sind durch den Schaft bis an das distale Ende 8 des Endoskops geführt, wo das am Lichtanschluss 12 eingekoppelte Beleuchtungslicht zur Beleuchtung eines endoskopischen Blickfelds austritt. Der Lichtanschluss ist dampfdicht an das Gehäuse 13 angesetzt. Das Gehäuse 13 ist ferner auch gegenüber dem Schaft 2 dampfdicht abgeschlossen, so dass der Innenraum 16 des Gehäuses 13 gemeinsam mit dem über den Metallbalg damit verbundenen Gehäuse 23 insgesamt dampfdicht abgeschlossen ist.

Innerhalb des Innenraums 16 geht das Hüllrohr, das in dem mit 50 bezeichneten Abschnitt beispielsweise aus PEEK bestehen kann, in einen Abschnitt 50' über, der beispielsweise aus einem metallischen Werkstoff bestehen kann und über einen kegelförmigen Abschnitt 50" in einen innerhalb des Schafts verlaufenden, ebenfalls aus einem metallischen Werkstoff bestehenden Abschnitt des Hüllrohrs übergeht. Die Abschnitte 50' und 50" sowie der innerhalb des Schafts verlaufende Abschnitt (nicht dargestellt) können insbesondere einstückig ausgebildet sein, während der Abschnitt 50 des Hüllrohrs dampfdicht mit dem Abschnitt 50' verbunden ist.

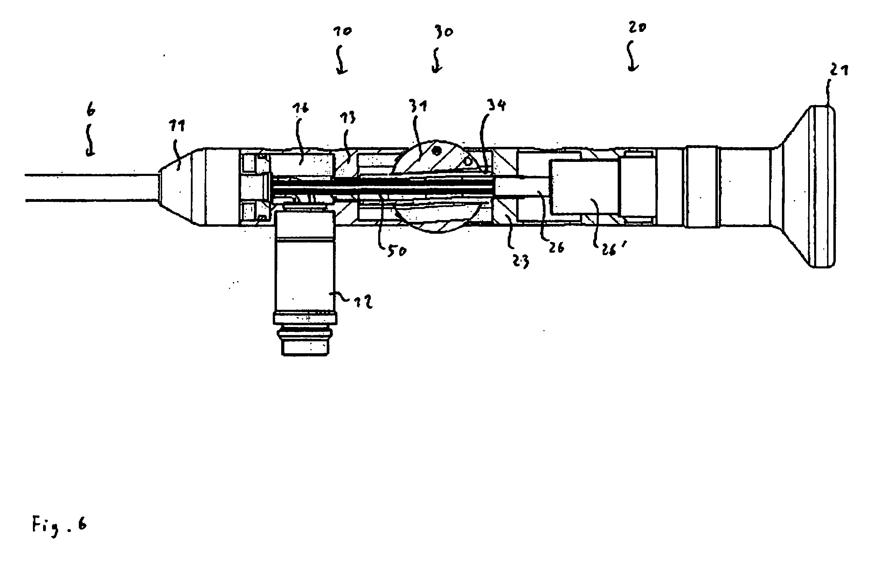

Fig. 6 zeigt in teilweise aufgeschnittener Darstellung eine weiteren Ausführungsform der Erfindung, bei der das Hüllrohr 50 insgesamt einstückig ausgebildet ist. Wie in Fig. 6 dargestellt, ist das Hüllrohr 50, vom Schaft 6 kommend, durch das erste Kopfteil 10 und das Gelenk 30 bis in das zweite Kopfteil 20 und in diesem in eine Führungshülse 26, 26' durchgeführt. Weitere Details und Bezugszeichen entsprechen denen des vorstehend beschriebenen Ausführungsbeispiels.

In einer nicht dargestellten weiteren Ausführungsform der Erfindung kann das zweite Kopfteil kann auch eine abgewinkelte Grundposition aufweisen, aus der heraus die Abwinkelung erfolgt; das erste Kopfteil kann in diesem Fall nach proximal auch über den Verbindungsbereich mit dem zweiten Kopfteil hinausreichen und beispielsweise in diesem Bereich einen Eingang für einen Instrumentenkanal aufweisen.

### Bezugszeichenliste

- 1: Endoskop
- 2: Schaft
- 3: Endoskopkopf
- 4: distaler Endbereich des Schafts
- 5: gekrümmter Abschnitt des Schafts
- 6: proximaler Abschnitt des Schafts
- 7: Markierung
- 8: distales Ende des Endoskops
- 9: Längsachse des proximalen Schaftbereichs
- 10: erstes Kopfteil
- 11: Konus
- 12: Lichtanschluss
- 13: Gehäuse des ersten Kopfteils
- 14: Bohrung
- 15: Längsachse des ersten Kopfteils
- 16: Innenraum
- 20: zweites Kopfteil
- 21: Okularmuschel
- 22: Griffmulde
- 23: Gehäuse des zweiten Kopfteils
- 24: Bohrung
- 25: Längsachse des zweiten Kopfteils
- 26, 26': Führungshülse
- 27: gestreckte Stellung
- 27': abgewinkelte Stellung
- 30: Gelenk
- 31: Gelenkscheibe
- 32: Bohrung
- 33: Drehpunkt
- 34: Metallbalg
- 35: distaler Endbereich des Metallbalgs
- 36: proximaler Endbereich des Metallbalgs
- 37: Stift
- 38: Scheibe
- 39: Anfasung
- 40: Aufnahme
- 41: Bohrung
- 42: Gleitscheibe
- 50, 50', 50": Abschnitte des Hüllrohrs
- 51: Halter
- 52: Zwischenraum
- 53: Bildleiter
- 54: Zwischenraum
- 60: Lichtleiter
- 61: Lichtleiter

## Patentansprüche

1. Endoskop, insbesondere zur Intubation eines Atemwegs, mit einem Schaft (2), der in seinem distalen Endbereich (4) ein Objektiv zur Aufnahme eines endoskopischen Bildes aufweist, und einem Endoskopkopf (3), der ein erstes Kopfteil (10), das mit dem Schaft (2) verbunden ist, sowie ein zweites Kopfteil (20), das ein Okular aufweist, umfast, wobei das zweite Kopfteil (20) abwinkelbar mit dem ersten Kopfteil (10) verbunden ist, und wobei das Endoskop zur Übertragung des vom Objektiv aufgenommenen Bildes zum Okular einen zumindest abschnittsweise flexiblen Bildleiter (53) umfasst, wobei ein Metallbalg (34) im Bereich der Verbindung zwischen erstem Kopfteil (10) und zweitem Kopfteil (20) angeordnet ist, **dadurch gekennzeichnet, dass** der Bildleiter (53) von einem vom Schaft bis zum zweiten Kopfteil durchgehenden, zumindest abschnittsweise biegeflexiblen Hüllrohr (50) umgeben ist, und dass das Hüllrohr (50) im Bereich eines Abwinkelungsabschnittes von dem Metallbalg (34) umgeben ist, wobei der Metallbalg (34) in seinem distalen Endbereich (35) dampfdicht mit einem Gehäuse (13) des ersten Kopfteils (10) verbunden ist.

2. Endoskop nach Anspruch 1, **dadurch gekennzeichnet, dass** das Hüllrohr (50) das Objektiv und/oder das Okular zumindest teilweise einschließt.

3. Endoskop nach Anspruch 1 oder 2, **dadurch gekennzeichnet, dass** das Hüllrohr (50) einteilig ausgebildet ist.

4. Endoskop nach Anspruch 1 oder 2, **dadurch gekennzeichnet, dass** das Hüllrohr (50) mehrteilig ausgebildet ist.

5. Endoskop nach einem der vorhergehenden Ansprüche, **dadurch gekennzeichnet, dass** der Metallbalg (34) in seinem proximalen Endbereich (36) dampfdicht mit einem Gehäuse (23) des zweiten Kopfteils verbunden ist.

6. Endoskop nach einem der vorhergehenden Ansprüche, **dadurch gekennzeichnet, dass** das zweite Kopfteil (20) über ein Gelenk (30) mit dem ersten Kopfteil (10) verbunden ist, das eine Abwinkelung des zweiten Kopfteils (20) in mindestens einer Ebene zulässt.

7. Endoskop nach Anspruch 6, **dadurch gekennzeichnet, dass** das Gelenk (30) derart ausgebildet ist, dass der Winkelbereich der Abwinkelung in der mindestens einen Ebene begrenzt ist.

8. Endoskop nach Anspruch 7, **dadurch gekennzeichnet, dass** der Winkelbereich der Abwinkelung asymmetrisch zu einer Längsachse (15) des ersten Kopfteils (10) angeordnet ist, insbesondere dass eine Abwinkelung des zweiten Kopfteils (20) aus einer gestreckten Position nur in eine Richtung möglich ist.

9. Endoskop nach einem der Ansprüche 6 bis 8, **dadurch gekennzeichnet, dass** das Gelenk (30) ein reibungserzeugendes Element umfasst.

10. Endoskop nach Anspruch 9, **dadurch gekennzeichnet, dass** die Reibung einstellbar ist.

11. Endoskop nach einem der Ansprüche 6 bis 10, **dadurch gekennzeichnet, dass** das Gelenk (30) eine Gelenkscheibe (31) umfasst, die eine Bohrung (32) zur Durchführung des den Bildleiter umgebenden Hüllrohrs (50) aufweist, wobei die Bohrung (32) exzentrisch angeordnet ist.

12. Endoskop nach einem der vorhergehenden Ansprüche, **dadurch gekennzeichnet, dass** das Hüllrohr (50) exzentrisch im Metallbalg (34) angeordnet ist.

## Claims

1. Endoscope, particularly for the intubation of an airway, with a shaft (2) which, in its distal end area (4), has a lens system for recording an endoscopic image, and with an endoscope head (3) comprising a first head part (10), which is connected to the shaft (2), and a second head part (20), which has an eyepiece, wherein the second head part (20) is connected pivotably to the first head part (10), and wherein the endoscope, in order to transmit the image recorded by the lens system to the eyepiece, comprises an image conductor (53) that is flexible at least in sections, and wherein a metal bellows (34) is arranged in the area of the connection between first head part (10) and second head part (20), **characterized in that** the image conductor (53) is surrounded by a protective tube (50) that runs continuously from the shaft as far as the second head part and is bendable at least in sections, and **in that** the protective tube (50) is surrounded by the metal bellows (34) in the area of a pivoting portion, wherein the metal bellows (34), in its distal end area (35), is connected to a housing (13) of the first head part (10) in a steam-tight manner.

2. Endoscope according to Claim 1, **characterized in that** the protective tube (50) at least partially encloses the lens system and/or the eyepiece.

3. Endoscope according to Claim 1 or 2, **characterized in that** the protective tube (50) is designed in one piece.

4. Endoscope according to Claim 1 or 2, **characterized in that** the protective tube (50) is designed in several pieces.

5. Endoscope according to one of the preceding claims, **characterized in that** the metal bellows (34), in its proximal end area (36), is connected to a housing (23) of the second head part in a steam-tight manner.

6. Endoscope according to one of the preceding claims, **characterized in that** the second head part (20) is connected to the first head part (10) via a joint (30) that permits a pivoting of the second head part (20) in at least one plane.

7. Endoscope according to Claim 6, **characterized in that** the joint (30) is designed in such a way that the angle range of the pivoting in the at least one plane is limited.

8. Endoscope according to Claim 7, **characterized in that** the angle range of the pivoting is asymmetrical with respect to a longitudinal axis (15) of the first head part (10), particularly **in that** a pivoting of the second head part (20) from a straight position is possible only in one direction.

9. Endoscope according to one of Claims 6 to 8, **characterized in that** the joint (30) comprises a friction-generating element.

10. Endoscope according to Claim 9, **characterized in that** the friction is adjustable.

11. Endoscope according to one of Claims 6 to 10, **characterized in that** the joint (30) comprises a joint disc (31), which has a bore (32) for the passage of the protective tube (50) surrounding the image conductor, the bore (32) being arranged eccentrically.

12. Endoscope according to one of the preceding claims, **characterized in that** the protective tube (50) is arranged eccentrically in the metal bellows (34).

## Revendications

1. Endoscope, en particulier pour l'intubation d'une voie respiratoire, comprenant une tige (2) qui présente, dans sa région d'extrémité distale (4), un objectif pour recevoir une image endoscopique, et une tête d'endoscope (3) qui comprend une première partie de tête (10) qui est connectée à la tige (2), ainsi qu'une deuxième partie de tête (20) qui présente un oculaire, la deuxième partie (20) étant connectée à la première partie de tête (10) de manière à pouvoir être fléchie, et l'endoscope comprenant, pour le transfert de l'image reçue par l'objectif à l'oculaire, un guide d'image (53) au moins partiellement flexible, un soufflet métallique (34) étant disposé dans la région de la connexion entre la première partie de tête (10) et la deuxième partie de tête (20), **caractérisé en ce que** le guide d'image (53) est entouré par un manchon tubulaire (50) au moins partiellement flexible, allant de la tige à la deuxième partie de tête, et **en ce que** le manchon tubulaire (50) est entouré, dans la région d'une portion coudée, par le soufflet métallique (34), le soufflet métallique (34) étant connecté dans sa région d'extrémité distale (35) de manière étanche à la vapeur par un boîtier (13) de la première partie de tête (10).

2. Endoscope selon la revendication 1, **caractérisé en ce que** le manchon tubulaire (50) renferme au moins en partie l'objectif et/ou l'oculaire.

3. Endoscope selon la revendication 1 ou 2, **caractérisé en ce que** le manchon tubulaire (50) est réalisé d'une seule pièce.

4. Endoscope selon la revendication 1 ou 2, **caractérisé en ce que** le manchon tubulaire (50) est réalisé en plusieurs parties.

5. Endoscope selon l'une quelconque des revendications précédentes, **caractérisé en ce que** le soufflet métallique (34) est connecté, dans sa région d'extrémité proximale (36) de manière étanche à la vapeur, à un boîtier (23) de la deuxième partie de tête.

6. Endoscope selon l'une quelconque des revendications précédentes, **caractérisé en ce que** la deuxième partie de tête (20) est connectée par le biais d'une articulation (30) à une première partie de tête (10), qui permet un coudage de la deuxième partie de tête (20) dans au moins un plan.

7. Endoscope selon la revendication 6, **caractérisé en ce que** l'articulation (30) est réalisée de telle sorte que la région coudée du coudage est limitée dans l'au moins un plan.

8. Endoscope selon la revendication 7, **caractérisé en ce que** la région angulaire du coudage est disposée sous forme asymétrique par rapport à un axe longitudinal (15) de la première partie de tête (10), en particulier **en ce qu'**un coudage de la deuxième partie de tête (20) à partir d'une position étirée n'est possible que dans une seule direction.

9. Endoscope selon l'une quelconque des revendications 6 à 8, **caractérisé en ce que** l'articulation (30) comprend un élément générateur de friction.

10. Endoscope selon la revendication 9, **caractérisé en ce que** la friction peut être ajustée.

11. Endoscope selon l'une quelconque des revendications 6 à 10, **caractérisé en ce que** l'articulation (30) comprend un disque d'articulation (31) qui présente un alésage (32) pour le passage du manchon tubulaire (50) entourant le guide d'image, l'alésage (32) étant disposé de manière excentrique.

12. Endoscope selon l'une quelconque des revendications précédentes, **caractérisé en ce que** le manchon tubulaire (50) est disposé de manière excentrique dans le soufflet métallique (34).
